# EUROPEAN PATENT SPECIFICATION

(11) **EP 3 034 162 B1**
(45) Date of publication and mention of the grant of the patent: **25.03.2020**
(21) Application number: 14836781.6
(22) Date of filing: 11.08.2014
(51) Int. Cl.: A61Q 5/00, A61Q 5/02, A61K 8/64, A61Q 19/10, B01J 13/02, A61Q 5/12

(54) **SURFACTANT COMPOSITION**
TENSIDZUSAMMENSETZUNG
COMPOSITION D'AGENT TENSIO-ACTIF

(30) Priority: 12.08.2013 JP 2013167765
(43) Date of publication of application: 22.06.2016
(73) Proprietor: Kaneka Corporation, Osaka-shi, Osaka 530-8288 (JP); National Institute of Advanced Industrial Science and Technology, Tokyo 100-8921 (JP)
(72) Inventor: YANAGISAWA, Satohiro, Tokyo 107-6025 (JP); IZUMIDA, Masashi, Takasago-shi Hyogo 676-8688 (JP); TAIRA, Toshiaki, Tsukuba-shi Ibaraki 305-8565 (JP); IMURA, Tomohiro, Tsukuba-shi Ibaraki 305-8565 (JP); KITAMOTO, Dai, Tsukuba-shi Ibaraki 305-8565 (JP)
(74) Representative: Hoffmann Eitle
(86) International application number: PCT/JP2014/071195
(87) International publication number: WO 2015/022936

(56) References cited:
- WO-A1-99/62482
- JP-A- H0 987 147
- JP-A- 2000 327 591
- JP-A- 2004 532 214
- JP-A- 2007 191 410
- JP-A- 2010 018 559
- YUKISHIGE KONDO ET AL: "Spontaneous Vesicle Formation from Aqueous Solutions of Didodecyldimethylammonium Bromide and Sodium Dodecyl sulfate Mixtures", LANGMUIR, vol. 11, no. 7, 1 July 1995 (1995-07-01), pages 2380-2384, XP055346721, US ISSN: 0743-7463, DOI: 10.1021/la00007a011
- BEHESHTEH SOHRABI ET AL: "Molecular Interactions of Cationic and Anionic Surfactants in Mixed Monolayers and Aggregates", JOURNAL OF PHYSICAL CHEMISTRY PART B: CONDENSED MATTER, MATERIALS, SURFACES, INTERFACES & BIOPHYSICAL, vol. 112, no. 47, 27 November 2008 (2008-11-27), pages 14869-14876, XP055347936, US ISSN: 1520-6106, DOI: 10.1021/jp803105n
- NOUDEH, G. D. ET AL.: 'The Effect of Temperature on Thermodynamic Parameters of Micellization of Some Surfactants' JOURNAL OF APPLIED SCIENCES vol. 7, no. 1, 2007, pages 47 - 52, XP008182772
- LIU, F. ET AL.: 'Interaction of the Biosurfactant, Surfactin with Betaines in Aqueous Solution' LANGMUIR vol. 29, 2013, pages 10648 - 10657, XP029671562
- YASUNARI NAKAMA: 'Kaimen Kasseizai - Ryoshin Baisei Kobunshi ga Hiraku Atarashii Oyo Gijutsu, Kaimen Kasseizai' RYOSHIN BAISEI KOBUNSHI NO SAISHIN KINO 30 June 2005, pages 117 - 130
- LI, Y. ET AL.: 'Counterion-Induced Changes to the Micellization of Surfactin-C16 Aqueous Solution' J. PHYS. CHEM. B vol. 113, 2009, pages 15272 - 15277, XP055316072

## Description

### TECHNICAL FIELD

The present invention relates to a surfactant composition which contains a micelle or a vesicle which is formed from an anionic surfactant and a cationic surfactant, and a hair cosmetic material which contains the surfactant composition.

### BACKGROUND ART

It is known that a cationic surfactant such as a quaternary ammonium salt and a tertiary ammonium salt has an effect of protecting hair by being adsorbed to a damaged portion of hair. On the one hand, a cationic surfactant has a problem of having significant skin irritancy.

In contrast, when an anionic surfactant and a cationic surfactant are used in combination, less skin irritation is caused as compared with the case of using a cationic surfactant only. There is therefore a possibility that a damaged portion of hair may be protected while suppressing skin irritation in such a case.

However, in particular, when an anionic surfactant and a cationic surfactant are used in combination in equimolar amounts or approximately equimolar amounts, both of the surfactants are strongly bonded to one another by electrostatic attractive force, leading to a problem of the formation and precipitation of an insoluble salt.

In the inventions of Patent Document 1 and Non-Patent Document 1, a specific quaternary ammonium salt is used as a cationic surfactant to achieve a solution for such a problem. In addition, in the invention of Patent Document 2, a vesicle which contains a ceramide and a cationic surfactant having two long-chain alkyl groups is utilized for a hair cosmetic material. A technique for forming a vesicle of a pseudo double chain-type surfactant by using a combination of an anionic surfactant and a cationic surfactant has been known before the prior arts.
WO 99 62482 discloses a surfactant for use in external preparations for skin including a lipopeptide compound derived from prokaryotes, an external preparation for skin containing such a surfactant, such as a cosmetic, and an external preparation for skin, such as a transparent cosmetic, e.g., a transparent cosmetic containing such a surfactant and a sequestering agent.

### PRIOR ART DOCUMENT

### PATENT DOCUMENT

Patent Document 1: JP 2011-131137 A
Patent Document 2: JP 2007-191410 A

Non-patent Document 1: ERIC W. KALER et al., SCIENCE, Vol. 245, pp. 1371-1374 (1989)

### DISCLOSURE OF THE INVENTION

### PROBLEMS TO BE SOLVED BY THE INVENTION

As described above, it has been known to use a surfactant composition which contains a vesicle for a hair cosmetic material. Patent Document 1 discloses also a problem that a cationic surfactant and an anionic surfactant are strongly bonded to form an insoluble salt.

However, when a conventional cationic surfactant and an anionic surfactant, of which hydrophilic group parts are small, are combined each other, a problem still exists that the cation and anion in the hydrophilic group parts are strongly bonded by electrostatic attractive force and hence an insoluble salt is formed. In addition, it is necessary to also suppress harm due to skin irritation or the like caused by a common surfactant.

Under such circumstances, an objective of the present invention is to provide a surfactant composition which hardly causes the formation of an insoluble salt and which contains a safe and extremely stable micelle or vesicle. Also, an objective of the present invention is to provide a hair cosmetic material which contains the surfactant composition.

### MEANS FOR SOLVING THE PROBLEMS

The inventors of the present invention made extensive studies to solve the above problems. As a result, the inventors completed the present invention by finding that an extremely stable micelle or vesicle is formed when surfactin, which is a natural surfactant, and a specific cationic surfactant are used in combination.

Hereinafter, the present invention is described.
[1] A surfactant composition,
   comprising a micelle or a vesicle,
   wherein the micelle or vesicle contains surfactin represented by the following formula (I): 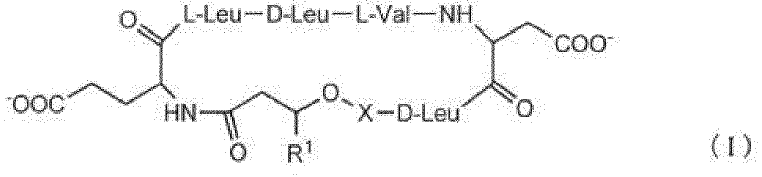 wherein 'X' is a residue of an amino acid selected from leucine, isoleucine and valine; and R¹ is a C₉₋₁₈ alkyl group, and a cationic surfactant having two C₈₋₂₀ alkyl groups or a cationic surfactant having one C₈₋₂₀ alkyl group;
   wherein the cationic surfactant having two C₈₋₂₀ alkyl groups is represented by the following formula (II): 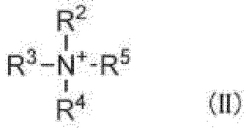 wherein R² and R³ are independently C₈₋₂₀ alkyl groups; R⁴ and R⁵ are independently C₁₋₄ alkyl groups; and R⁴ and R⁵ may bind to one another to form a ring;
   wherein the cationic surfactant having one C₈₋₂₀ alkyl group is represented by the following formula (III) or (IV): 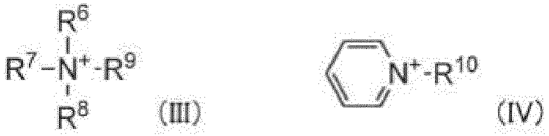 wherein R⁶ is a C₈₋₂₀ alkyl group; each of R⁷, R⁸ and R⁹ is independently C₁₋₄ alkyl groups; R¹⁰ is a C₈₋₂₀ alkyl group; and two of R⁷ to R⁹ may bind to one another to form a ring; and wherein the surfactant composition is defined further in claim 1.
[2] The surfactant composition according to the above [1], comprising the vesicle containing the surfactin (I) and the cationic surfactant having two C₈₋₂₀ alkyl groups. The cationic surfactant having two C₈₋₂₀ alkyl groups and the surfactin, which has one long-chain alkyl group, form a pseudo triple chain-type complex. Such a complex has not been known and may exhibit a behavior and physical properties which are different from those of a conventional pseudo double chain-type complex. In addition, a very stable vesicle may readily formed.
   In the surfactant composition according to the invention, the molar ratio of the cationic surfactant having two C₈₋₂₀ alkyl groups or cationic surfactant having one C₈₋₂₀ alkyl group to the surfactin (I) is not less than 0.8 and not more than 1.2. When the molar ratio is included in the above range, a micelle or vesicle can be formed more certainly. In addition, there is generally a problem that an insoluble salt is readily formed to be precipitated when an anionic surfactant and a cationic surfactant are used in combination in equimolar amounts or approximately equimolar amounts. However, in the case of the combination according to the present invention, even when such surfactants are used in equimolar amounts or approximately equimolar amounts, a very stable micelle or vesicle is formed and such an insoluble salt is hardly produced due to the unique bulky cyclic peptide structure of the surfactin (I). In addition, when the above-described molar ratio is included in the above range, skin irritation by a cationic surfactant can be inhibited and additionally, the effect to protect a damaged part of hair can be sufficiently obtained.
[3] The surfactant composition according to any one of the above [1] to [2], further comprising an aqueous solvent, and wherein the micelle or vesicle containing the surfactin (I) and the cationic surfactant having two C₈₋₂₀ alkyl groups or cationic surfactant having one C₈₋₂₀ alkyl group is dispersed in the aqueous solvent. The composition is extremely stable, since precipitate is hardly produced; therefore, the composition is very excellent as a product.
[4] A hair cosmetic material, comprising the surfactant composition according to any one of the above [1] to [3].

### EFFECT OF THE INVENTION

Since the surfactin (I), which is one component of the surfactant composition according to the present invention, has a bulky cyclic peptide portion, an insoluble salt is hardly formed and hence a micelle and a vesicle having a regular structure tend to be formed easily due to moderate electrostatic interaction of the surfactin (I) with a cationic surfactant. In addition, an alkyl chain tends to have a proper packing structure easily in such a micelle and vesicle. Thus, the micelle or vesicle which is contained in the surfactant composition according to the present invention is extremely stable, and a product which contains the surfactant composition according to the present invention has high stability. Furthermore, since the surfactin (I), which is one component of the surfactant composition according to the present invention, is safe due to low skin irritancy, the surfactant composition according to the present invention is also safe with low skin irritancy. For example, the surfactant composition according to the present invention is therefore useful as a safe hair cosmetic material having lower skin irritancy, since the cationic surfactant which is one component of the surfactant composition according to the present invention has not only a hair protective action as described above but also excellent in antibacterial activity.

In addition, the stable micelle and vesicle can respectively contain a hydrophobic or hydrophilic active ingredient or the like. The surfactant composition according to the present invention therefore can be used not only for a hair cosmetic material, a germicidal disinfectant or a disinfectant detergent but also for a drug delivery system, and is extremely useful for industries.

### BRIEF DESCRIPTION OF THE DRAWINGS

Fig. 1 are a differential interference microscope image and a polarizing microscope image of a vesicle which is composed of a surfactin/didecyldimethylammonium chloride composition.
Fig. 2 is a particle size distribution of a micelle which is composed of a surfactin/decyltrimethylammonium chloride composition.

### MODE FOR CARRYING OUT THE INVENTION

The surfactant composition according to the present invention is characterized in comprising the micelle or vesicle which contains the surfactin (I) and the specific cationic surfactant.

The surfactant composition according to the present invention essentially contains surfactin represented by the following formula (I). The surfactin (I) has a small environmental load and safety to a human body, since the surfactin (I) is a peptide compound. 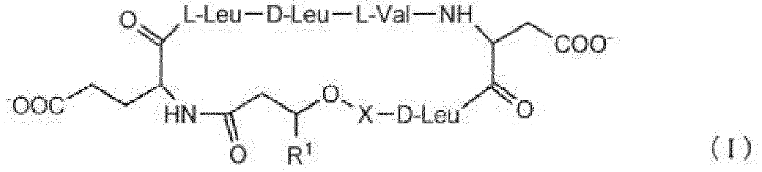 wherein 'X' is a residue of an amino acid selected from leucine, isoleucine and valine; and R¹ is a C₉₋₁₈ alkyl group.

Although the amino acid residue as 'X' may be either in a L-form or a D-form, the L-form is preferred.

The term "C₉₋₁₈ alkyl group" means a linear or branched monovalent saturated hydrocarbon group having not less than 9 and not more than 18 carbon atoms . The example thereof includes n-nonyl, 6-methyloctyl, 7-methyloctyl, n-decyl, 8-methylnonyl, n-undecyl, 9-methyldecyl, n-dodecyl, 10-methylundecyl, n-tridecyl, 11-methyldodecyl, n-tetradecyl, n-pentadecyl, n-hexadecyl, n-heptadecyl and n-octadecyl.

Either one of the surfactin (I) may be used or not less than two of the surfactin (I) may be used. For example, two or more surfactin (I) of which C₉₋₁₈ alkyl groups as R¹ are different may be used.

The surfactin (I) can be isolated from a culture broth prepared by culturing a microorganism such as a strain belonging to Bacillus subtilis in accordance with a known method. The surfactin (I) may be a purified product or an unpurified product. For example, a culture broth may be directly used as the unpurified product. Alternatively, the product of the surfactin (I) obtained by a chemical synthesis method may be similarly used.

The cationic surfactant which is an essential component of the surfactant composition according to the present invention has two C₈₋₂₀ alkyl groups or one C₈₋₂₀ alkyl group.

The term "C₈₋₂₀ alkyl group" means a linear or branched monovalent saturated hydrocarbon group having not less than 8 and not more than 20 carbon atoms. The example thereof includes n-octyl, isooctyl, 2-ethylhexyl, n-nonadecyl, isononadecyl, n-icosyl and isoicosyl in addition to the above-described C₉₋₁₈ alkyl group.

The C₈₋₂₀ alkyl group constitutes the hydrophobic portion of the cationic surfactant. When a cationic surfactant having two C₈₋₂₀ alkyl groups is used with the surfactin (I) in combination, the cationic group of the cationic surfactant and the anionic group of the surfactin (I) are bonded to one another by electrostatic attractive force to form a pseudo triple chain-type structure. The packing becomes dense and a strong and stable vesicle is formed due to such a pseudo triple chain-type structure in comparison with a general pseudo double chain-type structure. The vesicle coats hair surface in higher density due to the pseudo triple chain-type structure; as a result, tangle of hairs caused by electrostatic attractive force may be remarkably reduced. In addition, the vesicle may incorporate a water-soluble active ingredient or the like inside in an aqueous solvent dispersion.

On the one hand, when a cationic surfactant having one C₈₋₂₀ alkyl group is used with the surfactin (I) in combination, a pseudo double chain-type structure is obtained to form a micelle. A dispersion of general pseudo double chain-type is turbid; however, the micelle dispersion according to the present invention is transparent by visual observation. Although the reason is unknown, it is considered that very fine micelles are formed. The micelle may incorporate a hydrophobic active ingredient or the like inside in an aqueous solvent dispersion. In addition, the micelle is very useful as a formulation ingredient, since the micelle dispersion is transparent.

The cationic surfactant having two C₈₋₂₀ alkyl groups is represented by the following formula (II): 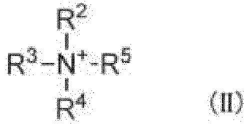 wherein R² and R³ are independently C₈₋₂₀ alkyl groups; R⁴ and R⁵ are independently C₁₋₄ alkyl groups; and R⁴ and R⁵ may bind to one another to form a ring.

The cationic surfactant having one C₈₋₂₀ alkyl group is represented by the following formula (III) or (IV): 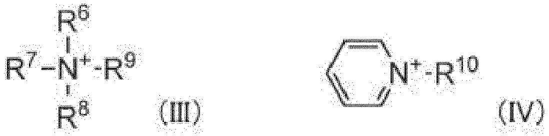 wherein R⁶ is a C₈₋₂₀ alkyl group; each of R⁷, R⁸ and R⁹ is independently C₁₋₄ alkyl groups; R¹⁰ is a C₈₋₂₀ alkyl group; and two of R⁷ to R⁹ may bind to one another to form a ring.

The term "C₁₋₄ alkyl group" means a linear or branched monovalent saturated hydrocarbon group having not less than 1 and not more than 4 carbon atoms. The example thereof includes methyl, ethyl, n-propyl, isopropyl, n-butyl, isobutyl, sec-butyl and t-butyl.

When the R⁴ and R⁵ or two of R⁷ to R⁹ bind to one another to form a ring, the ring is exemplified by a piperidine ring and a pyrrolidine ring.

As the cationic surfactant, a quaternary ammonium germicidal disinfectant such as benzalkonium chloride and benzethonium chloride may be used except for the above-described cationic surfactant.

Either one of the above-described cationic surfactants may be used or not less than two of the surfactants maybe used. In addition, the cationic surfactant having two C₈₋₂₀ alkyl groups and the cationic surfactant having one C₈₋₂₀ alkyl group may be used in combination. In other words, the term "a cationic surfactant having two C₈₋₂₀ alkyl groups or a cationic surfactant having one C₈₋₂₀ alkyl group" means one or more cationic surfactants selected from a cationic surfactant having two C₈₋₂₀ alkyl groups and a cationic surfactant having one C₈₋₂₀ alkyl group.

An amount of the surfactin (I) and the above-described cationic surfactant to be used may be appropriately adjusted in the range where a micelle or a vesicle is formed. The molar ratio of the cationic surfactant having two C₈₋₂₀ alkyl groups or cationic surfactant having one C₈₋₂₀ alkyl group to the surfactin (I) is not less than 0.8 and not more than 1.2.

Amounts of the surfactin (I) and the above-described cationic surfactant to be used are thus preferably equimolar or approximately equimolar each other. For example, when the above-described cationic surfactant in an excessive amount to the surfactin (I) is applied to a human body, skin irritation may be possibly caused. In addition, for example, when the surfactin (I) in an excessive amount to the above-described cationic surfactant is applied to hair, protective effect may not be possibly exerted to a satisfactory extent. On the one hand, when the above surfactants are used in equimolar amounts or approximately equimolar amounts, a micelle or vesicle is successfully formed and an effect by a cationic surfactant may be sufficiently exerted with inhibiting the above-described skin irritation. Thus, the molar ratio of the above-described cationic surfactant to the surfactin (I) is adjusted to not less than 0.8 and not more than 1.2.

The above-described surfactin (I) and cationic surfactant are bonded each other in an aqueous solvent to form a micelle or vesicle due to electrostatic attractive force.

The aqueous solvent is exemplified by water and a mixed solvent of water and a water-miscible solvent. The water-miscible solvent to be used in the present invention is exemplified by an alcohol solvent such as methanol, ethanol and isopropanol; an ether solvent such as tetrahydrofuran; an amide solvent such as dimethylformamide and dimethylacetamide; and a sulfoxide solvent such as dimethylsulfoxide. Since a water-miscible solvent may possibly inhibit the formation of a micelle and vesicle or cause skin irritation, a ratio of a water-miscible solvent to an aqueous mixed solvent is preferably not more than 50 mass%, more preferably not more than 25 mass%, even more preferably not more than 10 mass%, and particularly preferably not more than 5 mass%. The lower limit of the ratio is not particularly restricted, and may be not less than 0.5 mass%, preferably not less than 1 mass%, and more preferably not less than 2 mass%.

When a higher alcohol of which carbon number is not less than 6 and not more than 20 is added, the formation of a micelle or vesicle may be accelerated.

The surfactant composition according to the present invention can be produced without difficulty by mixing the surfactin (I) and the above-described cationic surfactant in an aqueous solvent. For example, the above-described cationic surfactant is mixed in an aqueous solution of the surfactin (I).

As the surfactin (I), a salt thereof may be used. Specifically, a salt of the surfactin (I) at least one of which two carboxy groups is electrostatically bound to an alkali metal ion or an ammonium ion can be used. Such a salt is very convenient due to a superior solubility in an aqueous solvent.

An alkali metal ion to be used for the salt of the surfactin (I) is not particularly restricted, and is exemplified by a lithium ion, a sodium ion, a potassium ion and the like. When two or more alkali metal ions are used, the ions are the same or different from each other.

A substituent of an ammonium ion is exemplified by a C₁₋₄ alkyl group such as methyl, ethyl, n-propyl, isopropyl, n-butyl and t-butyl; a C₇₋₁₄ aralkyl group such as benzyl, methylbenzyl and phenylethyl; and a C₆₋₁₂ aryl group such as phenyl, toluyl and xylyl. An ammonium ion is exemplified by a tetramethylammonium ion, a tetraethylammonium ion, a pyridinium ion and the like.

Two counter cations in the salt of the surfactin (I) may be the same or different from each other. In addition, one of the carboxy groups may be in the state of -COOH or -COO⁻.

The above-described cationic surfactant to be used may be in the condition of a salt. The counter anion thereof is exemplified by a halide ion such as a chloride ion, a bromide ion and an iodide ion; an inorganic acid ion such as a nitrate ion, a phosphate ion, a sulfate ion and a hydrogensulfate ion; and an organic acid ion such as an acetate ion, a trifluoroacetate ion, a methanesulfonate ion, a toluenesulfonate ion and a trifluoromethanesulfonate ion.

The concentration of the aqueous solvent solution of the salt of the surfactin (I) may be appropriately adjusted, and for example, may be adjusted to not less than about 1 mM and not more than 50 mM or not less than about 0.1 mass% and not more than about 5 mass%.

Then, the above-described cationic surfactant in an appropriate amount is added to be mixed in the aqueous solvent solution of the salt of the surfactin (I). The condition at the time may be appropriately selected, and for example, the temperature may be an ordinary temperature. Specifically, the temperature may be adjusted to not less than about 10°C and not more than about 50°C. In addition, in the present invention, the mixture may be moderately stirred, since a micelle or vesicle is easily formed without adding an external force such as ultrasonic waves. The stirring time may be adjusted to not less than about 10 minutes and not more than about 5 hours. In general, a micelle and vesicle are formed by adding an external force such as ultrasonic waves. However, in such a case, it is very difficult to produce a micelle and vesicle which have even particle size and which is excellent in dispersion stability with high reproducibility. On the one hand, the vesicle and micelle according to the present invention is thermodynamically stable and excellent in dispersion stability, and the particle diameter thereof is narrow, since the vesicle and micelle can be produced by moderate stirring treatment without an external force. In addition, a vesicle and micelle which have the same characteristics can be produced at high reproducibility.

The surfactin composition according to the present invention can be applied to various products which are exemplified by a hair cosmetic material such as a hair conditioner, a hair treatment product, a hair pack, a hair cream and a hair lotion; a sterilizing disinfectant; and a sterilizing detergent. In such a product, the dispersion of the obtained above-described micelle or vesicle may be concentrated, and other component may be added depending on the use application.

The other component is not particularly restricted, and is exemplified by a polyol such as ethylene glycol, propylene glycol, glycerin and sorbitol; a polysaccharide thickener such as guar gum and xanthane gum; a cellulose compound such as hydroxypropyl cellulose and carboxymethyl cellulose; a carboxyvinyl polymer such as an acrylic acid polymer and an acrylic acid copolymer; a silicone compound; a non-ionic surfactant solution; a colorant; a pH adjuster; a plant extract; a preservative; a chelating agent; a vitamin preparation; a medicinal ingredient such as an anti-inflammatory drug; a fragrance; a ultraviolet absorber; an antioxidant; a sphingolipid such as ceramide; an oil such as olive oil, coconut oil, palm oil and canola oil.

### EXAMPLES

Hereinafter, the present invention is described in more detail with Examples. However, the present invention is not restricted to the following Examples in any way, and it is possible to work the present invention according to the Examples with an additional appropriate change within the range of the above descriptions and the following descriptions. Such a changed embodiment is also included in the technical scope of the present invention.

### Example 1: Preparation of pseudo triple chain-type surfactant vesicle

Surfactin sodium salt having C₉₋₁₈ long-chain alkyl groups was dissolved in ultrapure water to produce 19 mM solution (5 mL). The concentration corresponds to about 2 mass%. Hereinafter, surfactin sodium salt is referred to as "SFNa". Into the solution, a cationic surfactant having two long-chain alkyl groups was added in an equimolar. The mixture was moderately stirred at room temperature for 1 hour without adding an external force such as ultrasonic waves. Then, the reaction mixture was observed. As the cationic surfactant having two long-chain alkyl groups, dimethyldidodecylammonium chloride, dimethyldidecylammonium chloride and dimethyldioctylammonium chloride were used. The result is shown in Table 1.

**[Table 1]**

| Composition No. | Composition | Structure | Condition | Viscosity |
|---|---|---|---|---|
| 1 | SFNa/dimethyldidodecylammonium chloride | pseudo triple chain-type | white/turbid | low |
| 2 | SFNa/dimethyldidecylammonium chloride | pseudo triple chain-type | white/turbid | low |
| 3 | SFNa/dimethyldioctylammonium chloride | pseudo triple chain-type | white/turbid | low |

### Test example 1: Stability test

The dispersions of the pseudo triple chain-type surfactant vesicle obtained in Example 1 were turbid in white. The dispersions were left to stand at room temperature for 4 months. As a result, it was demonstrated that molecular aggregates which have high stability were formed in all of the dispersions, since the formation of precipitate was not observed.

### Test example 2: Observation of condition

As the above-described Example 1, SFNa was dissolved in ultrapure water to produce 4.8 mM solution (5 mL). The concentration corresponds to about 0.5 mass%. Into the solution, dimethyldidecylammonium chloride was added in an equimolar. The mixture was moderately stirred at room temperature for 1 hour to produce a white dispersion.

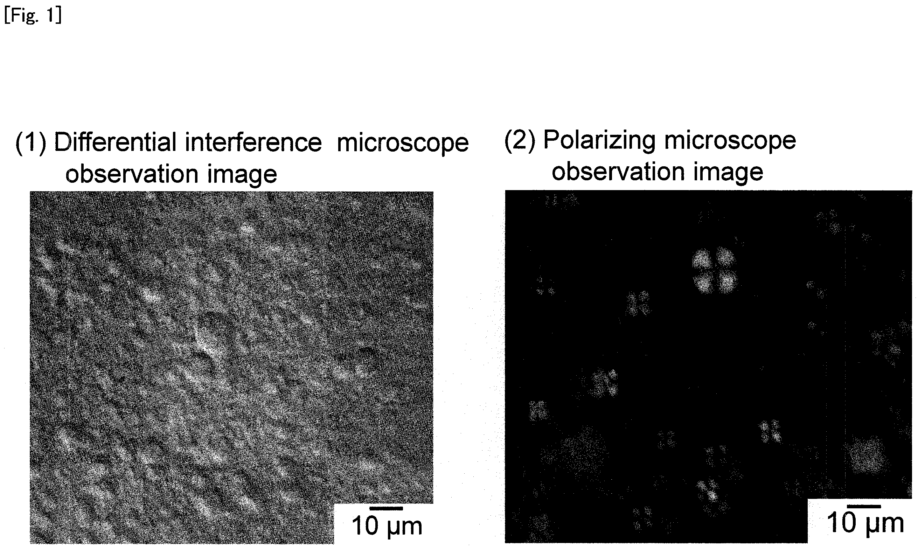

The dispersion was thinly spread on a preparation to be observed by a polarizing microscope (product name: ECLIPSE E600, manufactured by Nikon Corporation) equipped with a CCD camera (product name: DS-5M, manufactured by Nikon Corporation). Also, differential interference observation was carried out using the microscope. The result of differential interference microscope observation is shown in Figure 1(1), and the result of polarizing microscope observation is shown in Figure 1(2).

As Figure 1, when the sample was observed using a differential interference microscope, particles having diameters of several micrometers to dozens of micrometers were confirmed. When the same sample was observed using a polarizing microscope, Maltese cross was observed under crossed Nicols condition. Thus, it was demonstrated that a vesicle was formed.

### Example 2: Preparation of pseudo double chain-type surfactant micelle

A SFNa/cationic surfactant composition was prepared similarly to the above-described Example 1 except that a cationic surfactant having one long-chain alkyl group was used in place of a cationic surfactant having two long-chain alkyl groups. As the cationic surfactant having one long-chain alkyl group, dodecyltrimethylammonium chloride, decyltrimethylammonium chloride and octyltrimethylammonium chloride were used. The observation result of the characteristics is shown in Table 2.

**[Table 2]**

| Composition No. | Composition | Structure | Condition | Viscosity |
|---|---|---|---|---|
| 4 | SFNa/dodecyltrimethylammonium chloride | pseudo double chain-type | colorless/transparent | high |
| 5 | SFNa/decyltrimethylammonium chloride | pseudo double chain-type | colorless/transparent | low |
| 6 | SFNa/octyltrimethylammonium chloride | pseudo double chain-type | colorless/transparent | low |

In particular, in the case of the combination of SFNa/dodecyltrimethylammonium chloride, it was suggested that a string-like micelle was formed since the obtained solution was transparent and highly viscous.

### Test example 3: Stability test

The dispersions of the pseudo double chain-type surfactant micelle obtained in Example 2 were colorless and transparent. The dispersions were left to stand at room temperature for 3 months. As a result, it was demonstrated that molecular aggregates which have high stability were formed in all of the dispersions, since the formation of precipitate was not observed.

### Test example 4: Confirmation of micelle formation

As the above-described Example 1, SFNa was dissolved in ultrapure water to produce 19 mM solution (5 mL). The concentration corresponds to about 2 mass%. Into the solution, decyltrimethylammonium chloride was added in an equimolar. The mixture was moderately stirred at room temperature for 1 hour.

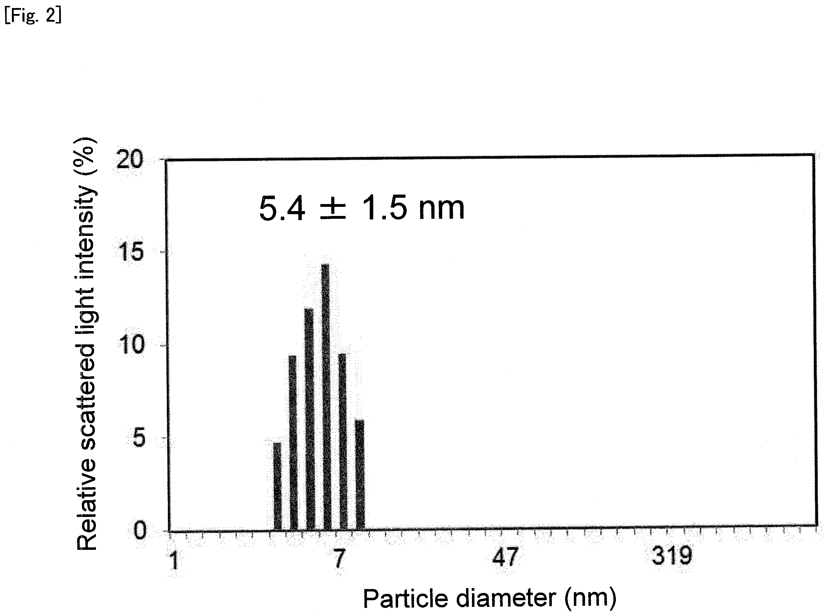

The obtained transparent dispersion (5 mL) was added into a glass cell, and the size distribution of the particles which were contained in the dispersion was measured by a dynamic light scattering measuring device (product name: DLS-7000, manufactured by OTSUKA ELECTRONICS Co., LTD.). Ar laser of which λ was 488 nm was used as a light source, and the scattering angle was adjusted to 90°. The result is shown in Figure 2.

As Figure 2, it was demonstrated that micelles of which average particle diameter was 5.4±1.5 nm was formed.

## Claims

1. A surfactant composition,
comprising a micelle or a vesicle,
wherein the micelle or vesicle contains surfactin represented by the following formula (I): 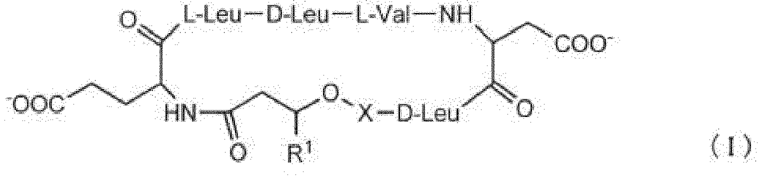
wherein 'X' is a residue of an amino acid selected from leucine, isoleucine and valine; and R¹ is a C₉₋₁₈ alkyl group,
and a cationic surfactant having two C₈₋₂₀ alkyl groups or a cationic surfactant having one C₈₋₂₀ alkyl group,
wherein the molar ratio of the cationic surfactant having two C₈₋₂₀ alkyl groups or cationic surfactant having one C₈₋₂₀ alkyl group to the surfactin (I) is not less than 0.8 and not more than 1.2,
wherein the cationic surfactant having two C₈₋₂₀ alkyl groups is represented by the following formula (II): 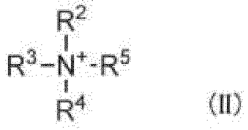
wherein R² and R³ are independently C₈₋₂₀ alkyl groups; R⁴ and R⁵ are independently C₁₋₄ alkyl groups; and R⁴ and R⁵ may bind to one another to form a ring, and
wherein the cationic surfactant having one C₈₋₂₀ alkyl group is represented by the following formula (III) or (IV): 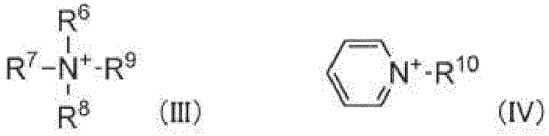
wherein R⁶ is a C₈₋₂₀ alkyl group; each of R⁷, R⁸ and R⁹ is independently a C₁₋₄ alkyl group; R¹⁰ is a C₈₋₂₀ alkyl group; and two of R⁷ to R⁹ may bind to one another to form a ring.

2. The surfactant composition according to claim 1, comprising the vesicle containing the surfactin (I) and the cationic surfactant having two C₈₋₂₀ alkyl groups.

3. The surfactant composition according to claim 1 or 2, further comprising an aqueous solvent, and wherein the micelle or vesicle containing the surfactin (I) and the cationic surfactant having two C₈₋₂₀ alkyl groups or cationic surfactant having one C₈₋₂₀ alkyl group is dispersed in the aqueous solvent.

4. A hair cosmetic material, comprising the surfactant composition according to any one of claims 1 to 3.

## Patentansprüche

1. Tensidzusammensetzung,
umfassend eine Mizelle oder ein Vesikel,
wobei die Micelle oder das Vesikel Surfactin, dargestellt durch die folgende Formel (I): 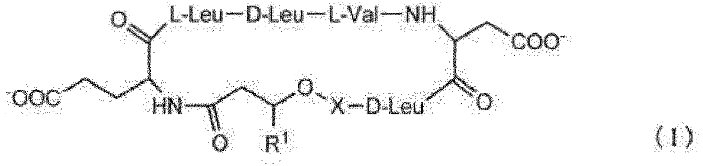
worin "X" ein Rest einer Aminosäure, ausgewählt aus Leucin, Isoleucin und Valin, ist und R¹ eine C₉₋₁₈-Alkylgruppe ist,
und ein kationisches Tensid mit zwei C₈₋₂₀-Alkylgruppen oder ein kationisches Tensid mit einer C₈₋₂₀-Alkylgruppe enthält,
wobei das Molverhältnis des kationischen Tensids mit zwei C₈₋₂₀-Alkylgruppen oder kationischen Tensids mit einer C₈₋₂₀-Alkylgruppe zu dem Surfactin (I) nicht weniger als 0,8 und nicht mehr als 1,2 beträgt,
wobei das kationische Tensid mit zwei C₈₋₂₀-Alkylgruppen durch die folgende Formel (II) dargestellt ist: 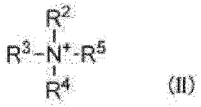
worin R² und R³ unabhängig C₈₋₂₀-Alkylgruppen sind, R⁴ und R⁵ unabhängig C₁₋₄-Alkylgruppen sind und R⁴ und R⁵ aneinander binden sein können, um einen Ring zu bilden, und
wobei das kationische Tensid mit einer C₈₋₂₀-Alkylgruppe durch die folgende Formel (III) oder (IV) dargestellt ist: 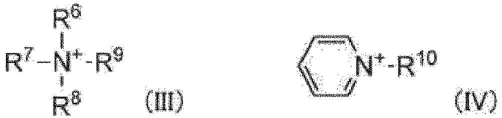
worin R⁶ eine C₈₋₂₀-Alkylgruppe ist, jedes von R⁷, R⁸ und R⁹ unabhängig eine C₁₋₄-Alkylgruppe ist, R¹⁰ eine C₈₋₂₀-Alkylgruppe ist und zwei von R⁷ bis R⁹ aneinander binden können, um einen Ring zu bilden.

2. Tensidzusammensetzung gemäß Anspruch 1, umfassend das Vesikel, das das Surfactin (I) und das kationische Tensid mit zwei C₈₋₂₀-Alkylgruppen enthält.

3. Tensidzusammensetzung gemäß Anspruch 1 oder 2, ferner umfassend ein wässriges Lösungsmittel und wobei die Mizelle oder das Vesikel, die/das das Surfactin (I) und das kationische Tensid mit zwei C₈₋₂₀-Alkylgruppen oder das kationische Tensid mit einer C₈₋₂₀-Alkylgruppe enthält, in dem wässrigen Lösungsmittel dispergiert ist.

4. Haarkosmetisches Material, umfassend die Tensidzusammensetzung gemäß irgendeinem der Ansprüche 1 bis 3.

## Revendications

1. Composition d'agent tensioactif,
comprenant une micelle ou un liposome,
dans laquelle la micelle ou le liposome contient de la surfactine représentée par la formule (I) suivante : 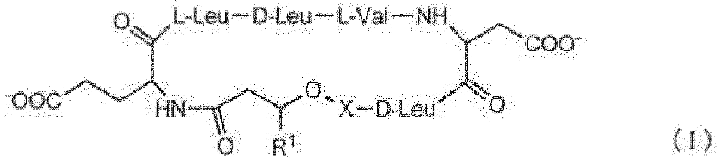
dans laquelle « X » est un résidu d'un acide aminé sélectionné parmi la leucine, l'isoleucine et la valine ; et R¹ est un groupe alkyle en C₉₋₁₈,
et un agent tensio-actif cationique ayant deux groupes alkyle en C₈₋₂₀ ou un agent tensio-actif cationique ayant un groupe alkyle en C₈₋₂₀,
dans laquelle le rapport molaire de l'agent tensio-actif cationique ayant deux groupes alkyle en C₈₋₂₀ ou de l'agent tensio-actif cationique ayant un groupe alkyle en C₈₋₂₀ sur la surfactine (I) n'est pas inférieur à 0,8 et pas supérieur à 1,2,
dans laquelle l'agent tensio-actif cationique ayant deux groupes alkyle en C₈₋₂₀ est représenté par la formule (II) suivante : 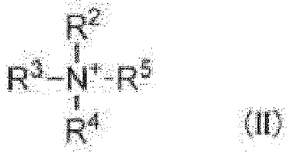
dans laquelle R² et R³ sont indépendamment des groupes alkyle en C₈₋₂₀ ; R⁴ et R⁵ sont indépendamment des groupes alkyle en C₁₋₄; et R⁴ et R⁵ peuvent se lier l'un à l'autre pour former un cycle, et
dans laquelle l'agent tensio-actif cationique ayant un groupe alkyle en C₈₋₂₀ est représenté par la formule (III) ou (IV) suivante : 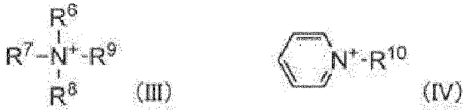
dans laquelle R⁶ est un groupe alkyle en C₈₋₂₀ ; chacun de R⁷, R⁸ et R⁹ est indépendamment un groupe alkyle en C₁₋₄ ; R¹⁰ est un groupe alkyle en C₈₋₂₀ ; et deux de R⁷ à R⁹ peuvent se lier l'un à l'autre pour former un cycle.

2. Composition d'agent tensio-actif selon la revendication 1, comprenant le liposome contenant la surfactine (I) et l'agent tensio-actif cationique ayant deux groupes alkyle en C₈₋₂₀.

3. Composition d'agent tensio-actif selon la revendication 1 ou 2, comprenant en outre un solvant aqueux, et dans laquelle la micelle ou le liposome contenant la surfactine (I) et l'agent tensio-actif cationique ayant deux groupes alkyle en C₈₋₂₀ ou l'agent tensioactif cationique ayant un groupe alkyle en C₈₋₂₀ est dispersé dans le solvant aqueux.

4. Matériau cosmétique pour les cheveux, comprenant la composition d'agent tensio-actif selon l'une quelconque des revendications 1 à 3.
